Europäisches Patentamt

European Patent Office

Office européen des brevets

(19)

(11) Numéro de publication: **0 093 128 B1**

(12) **FASCICULE DE BREVET EUROPEEN**

(45) Date de publication du fascicule du brevet:
**21.08.85**

(21) Numéro de dépôt: **82903246.5**

(22) Date de dépôt: **05.11.82**

(86) Numéro de dépôt international:
**PCT/FR 82/00181**

(87) Numéro de publication internationale:
**WO 83/01619 (11.05.83 Gazette 83/11)**

(51) Int. Cl.⁴: **C 07 K 1/10**, C 07 K 1/12,
C 07 K 1/14, C 07 K 5/06,
A 23 L 1/236

(54) PROCEDE DE PREPARATION DE L'ESTER-METHYLIQUE DE L'ALPHA-ASPARTYL PHENYLALANINE.

(30) Priorité: **06.11.81 FR 8120877**

(43) Date de publication de la demande:
**09.11.83 Bulletin 83/45**

(45) Mention de la délivrance du brevet:
**21.08.85 Bulletin 85/34**

(84) Etats contractants désignés:
**AT BE CH DE FR GB LI LU NL SE**

(56) Documents cités:
**CHEMICAL ABSTRACTS, Vol. 86, No. 21, 23 May 1977
(Columbus, Ohio, US), see page 505, abstract No.
155982W, JP, A, 76113841, 07 October 1976, Ajinomoto
Co. Inc.
JOURNAL OF THE CHEMICAL SOCIETY, 1955 A.R.
Battersby et al.: "Studies on specific chemical fission of
peptide links. Part I. The rearrangement of aspartyl and
glutamyl peptides", see pages 259-269**

(73) Titulaire: **LABORATOIRES FOURNIER S.A., 9 rue Petitot,
F-21100 Dijon (FR)**

(72) Inventeur: **GOURBAULT, Maurice Jean, 11 Rue de
Lubeck, 75116 Paris (FR)**

(74) Mandataire: **Combe, André, CABINET BEAU DE
LOMENIE 55 rue d'Amsterdam, F-75008 Paris (FR)**

BUNDESDRUCKEREI BERLIN

## Description

La présente invention concerne un procédé de préparation de l'ester méthylique de l'α-aspartyl phénylalanine.

On sait que l'ester méthylique de l'α-aspartyl phénylalanine (ou aspartam) est un édulcorant artificiel acalorique très recherché. Ses propriétés pharmacologiques et toxicologiques sont maintenant bien connues et son utilisation ne comporte aucun des risques que l'on attribue à l'utilisation des produits tels que la saccharine et le cyclohexylsulfamate.

On sait également que cet aspartam doit être utilisé sous forme de son isomère α car son isomère β est dépourvu de pouvoir sucrant et est doué, au contraire, d'une saveur amère.

Il est donc souhaitable de disposer d'un procédé de synthèse simple de cet aspartam, ce procédé permettant de minimiser la production de produits secondaires indésirables (et notamment de cet isomère β) et comportant une technique de valorisation par transformation de cet isomère β en isomère α.

Le procédé utilisé consiste a effectuer en milieu basique aqueux, et de façon connue, la condensation de l'ester-méthylique de la phénylalanine avec l'anhydride de l'acide N-formyl L-aspartique et est caractérisé en ce que le produit de ladite réaction de condensation est traité dans au moins une colonne de résine échangeuse d'ions dans laquelle se réalise notamment l'acidification dudit produit et qu'après récupération de l'isomère α N formylé d'une part et d'un mélange d'isomère α et d'isomère β N formylés d'autre part, on traite ledit mélange des isomères α et β par l'anhydride acétique de façon obtenir l'aspartimide correspondat puis le produit obtenu par un acide de façon à transformer en isomère α la plus grande partie de l'isomère β contenu dans ledit mélange et à assurer la déformylation de ces produits.

Comme il a été précisé la réaction de condensation de l'esterméthylique de la phénylalanine avec l'anhydride de l'acide N-formyl L-aspartique est connue. Elle est connue d'une part du fait qu'elle ne constitue qu'on emploi nouveau d'un procédé général de condensation de deux aminoacides et d'autre part du fait qu'elle a été décrite dans un certain nombre de documents parmi lesquels les brevets français 2 040 473, 2 078 640 et 2 160 471.

Cependant, les techniques par lesquelles, à la fin de la réaction de condensation décrite ci-dessus, on récupère les produits formés en vue de la préparation de l'isomère α sur de l'aspartam ont une impotance considérable pour la rentabilité dudit procédé et pour la possibilité d'obtention d'un produit final aussi pur que possible.

Dans la présente invention le produit de réaction est donc soumis à une acidification par passage dans une colonne échangeuse d'ions contenant une résine de type polystyrène sulfonique. Ce passage a pour résultat d'une part de convertir les sels de sodium formés dans la réaction de condensation en acides correspondant d'autre part de réaliser une déminéralisation du milieu. On peut obtenir ainsi de façon très courante un produit (aspartam comportant un blocage formyl) contenant plus d'environ 80% d'isomère α de N-formyl aspartam; la teneur de l'éluat en cet isomère α est telle qu'il est possible, après concentration, de précipiter un produit cristallin solide par simple ensemencement de l'éluat concentré à l'aide de cristaux de l'isomère α du N-formyl aspartam.

On voit l'intérêt d'un tel traitement.

Le N-formyl aspartam de forme α ainsi obtenu peut être déformylé par des techniques, par exemple par traitement à l'acide chlorhydrique dilué à des températures comprises entre 30 et 75° C, de façon à obtenir l'aspartam.

Mais par ailleurs le procédé de condensation a donné naissance à du N-formyl aspartam de forme β qui est récupéré tel que ou qui le plus souvent se présente sous forme de mélanges avec le N-formyl aspartam de forme α. Il est donc souhaitable de disposer d'un procédé permettant de transformer cet isomère β en isomère α.

Selon la présente invention on réalise simultanément l'isomérisation de l'isomère β en isomère α et la déformylation du mélange de départ en réalisant la transformation décrite ci-après. Ainsi la présente invention concerne un procédé pour la préparation de l'ester méthylique de l'α-aspartyl phénylalanine selon lequel on effectue en milieu basique aqueux, et de façon connue, la condensation de l'ester méthylique de la phénylalanine avec l'anhydride de l'acide N-formyl-L-aspartique, ledit procédé étant caractérisé en ce que le produit de ladite réaction de condensation est traité dans au moins une colonne de résine échangeuse d'ions dans laquelle se réalise notamment l'acidification dudit produit et qu'après récupération de l'isomère α-N-formylé d'une part et d'un mélange d'isomère α et d'isomère β-N-formylés d'autre part, on réalise les deux stades suivants:

— traitement dudit isomère β ou du mélange dudit isomère β et dudit isomère α en solution dans l'anhydride acétique à une température comprise entre 10 et 70° C en vue de la formation d'un aspartimide;
— puis on soumet ledit aspartimide à une hydrolyse en solution acide à pH comris entre 2,5 et 3,5;

après quoi, la solution obtenue est soumise à cristallisation.

Ladite hydrolyse acide de l'aspartimide peut être réalisée par addition d'acide chlorhydrique en solution dans le méthanol. Les exemples non limitatifs suivants illustrent les divers aspects de l'invention.

## Exemple 1

Traitement du produit de condensation de l'ester méthylique de la phénylalanine avec l'anhydride de l'acide N-formyl aspartique.

On a réalisé la réaction de condensation en utilisant des quantités sensiblement équivalentes, en poids, des deux réactifs et une solution aqueuse de soude. Le pH est ajusté à 8,5 et la température à 0°C. La réaction dure deux heures environ.

La solution froide obtenue est percolée sur une colonne de résine duolite® 0—20 sulfonique; le volume de la colonne est proportionnel aux quantités à traiter et le débit d'élution est déter-mimé par des solutions.

On obtient un percolat très acide dont on ajuste le pH à environ 2,4 par additon de chaux; on filtre le sulfate de calcium précipité. Le filtrat est à nouveau percolé sur cette même colonne et dans les mêmes conditions pour notamment en éliminer le sodium non fixé et le calcium restant.

Le percolat est concentré sous vide.

On obtient une solution qui, par simple adjonction de cristaux de N-formyl α aspartam, cristallise.

Les cristaux obtenus contiennent au moins 80% de l'isomère α du N-formyl aspartam, et sont purifiables par recristallisation.

## Exemple 2

Isomérisation et déformylation de mélange d'isomère α et β de N-formyl-aspartam.

En effectuant la réaction de condensation décrite dans l'exemple 1 on a obtenu également une solution contenant en proportions sensiblement analogues de l'isomères α du N-formyl aspartam et de l'isomère β de ce même produit.

Cette solution a été traitée comme suit:

— on admet dans un réacteur environ 2 kg de ce précipité et 10 kg d'anhydride acétique; on porte le mélange à 60°C pendant une heure; on concentre sous vide jusqu'à l'obtention d'un précipité ou d'une huile;
— le précipité (ou l'huile) obtenu est dissout dans le méthanol et on y ajoute une quantité suffisante d'acide chlorhydrique pour que le pH du milieu soit compris entre 2,5 et 3,5 environ; on chauffe pendant une heure à 60°C:
— par cristallisation on obtient directement de l'isomère α d'aspartam. Dans ces réactions on a réalisé simultanément l'isomérisation de l'isomère β du N-formyl aspartam et la déformylation de cet isomère.

## Revendications

1. Procédé pour la préparation de l'ester méthylique de l'α-aspartyl phénylalanine selon lequel on effectue en milieu basique aqueux, et de façon connue, la condensation de l'ester méthylique de la phénylalanine avec l'anhydride de l'acide N-formyl-L-aspartique, ledit procédé étant caractérisé en ce que le produit de ladite réaction de condensation est traité dans au moins une colonne de résine échangeuse d'ions contenant une résine de type polystyrène sulfonique dans laquelle se réalise notamment l'acidifiaction dudit produit et qu'après récupération de l'isomère α-N-formylé d'une part et d'un mélange d'isomère β-N-formylés d'autre part, on réalise les deux stades suivants:

— traitement dudit isomère β ou du mélange dudit isomère β et dudit isomère α en solution dans l'anhydride acétique à une température comprise entre 10 et 70°C en vue de la formation d'un aspartimide;
— puis on soumet ledit aspartimide à une hydrolyse en solution acide à pH compris entre 2,5 et 3,5, après quoi la solution obtenue est soumise à cristallisation.

2. Procédé selon la revendication 1, caractérisé en ce que l'hydrolyse acide de l'aspartimide est réalisée au moyen d'acide chlorhydrique en solution dans le méthanol.

## Patentansprüche

1. Verfahren zur Herstellung von α-Asparaginyl-phenylalanin-methylester, bei welchem in wässerigem basischem Milieu und auf bekannte Weise die Kondensation von Phenylalanin-methylester mit dem N-Formyl-L-asparaginsäureanhydrid durchgeführt wird, dadurch gekennzeichnet, daß das Produkt der Kondensationsreaktion in mindestens einer ein Harz des Polystyrolsulfontyps enthaltenden Ionenaustauscherharzsäule behandelt wird, in welcher insbesondere die Säurebildung des Produkts vor sich geht, und daß nach der Gewinnung des N-formylierten α-Isomeren einerseits und einer Mischung aus N-formylierten α- und β-Isomeren andererseits folgende zwei Stufen ausgeführt werden:

— Behandlung des β-Isomeren oder der Mischung des β-Isomeren und des α-Isomeren in Lösung in Essigsäureanhydride bei einer Temperatur zwischen 10 und 70°C zur Bildung eines Asparaginsäureimids,
— anschließende Hydrolyse des Asparaginsäureimids in saurer Lösung bei einem pH-Wert zwischen 2,5 und 3,5, worauf die erhaltene Lösung kristallisieren gelassen wird.

2. Verfahren nach Anspruch 1, dadurch gekennzeichnet, daß die saure Hydrolyse des Asparaginsäureimids mittels in Methanol gelöster Salzsäure durchgeführt wird.

## Claims

1. Process for the preparation of $\alpha$-aspartyl phenylalanine methyl ester consisting in condensing in known manner in an aqueous base medium, phenylalanine methyl ester with the anhydride of N-formyl L-aspartic acid, said process being characterized in that the product from said condensation reaction is treated in at least one ion-exchanging resin column containing a resin of the sulphonic polystyrene type in which acidification of said product takes place and in that after recovery of the N-formyl $\alpha$-isomer on the one hand, and of a mixture of N-formyl $\alpha$- and $\beta$-isomers, on the other hand, the two following steps are carried out:

— treatment of said $\beta$-isomer or of the mixture of said $\beta$-isomer and said $\alpha$-isomer with acetic anhydride in solution at a temperature varying between about 10 and 70° C to form an aspartimide;
— said aspartimide is subjected to a hydrolisis in acid solution at a pH varying between 2.5 and 3.5, the solution obtained is then subjected to a crystallization.

2. Process according to claim 1, characterized in that the acid hydrolysis of aspartimide is carried out by means of hydrochloric acid in solution in the methanol.